(19) Europäisches Patentamt
European Patent Office
Office européen des brevets

(11) **EP 4 389 797 A1**

(12) **EUROPEAN PATENT APPLICATION**

(43) Date of publication:
26.06.2024 Bulletin 2024/26

(21) Application number: 22214780.3

(22) Date of filing: 20.12.2022

(51) International Patent Classification (IPC):
**C08G 69/10** (2006.01)     **A61F 13/49** (2006.01)
**C08G 69/48** (2006.01)

(52) Cooperative Patent Classification (CPC):
**C08G 69/10; C08G 69/48;** A61F 2013/530481;
C08L 2201/08; C08L 2203/02

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Evonik Superabsorber GmbH**
**45128 Essen (DE)**

(72) Inventors:
• **HENN, Markus**
**45894 Gelsenkirchen (DE)**

• **LOICK, Christoph**
**47199 Duisburg (DE)**
• **TENI, Rainer**
**47447 Moers (DE)**
• **HAGER, Marc**
**67500 Haguenau (FR)**

(74) Representative: **Evonik Patent Association**
**c/o Evonik Industries AG**
**IP Management**
**Bau 1042A/PB 15**
**Paul-Baumann-Straße 1**
**45772 Marl (DE)**

(54) **BIOLOGICALLY DEGRADABLE SUPERABSORBER**

(57)    The present invention relates to a biologically degradable superabsorber based on polyglutamic acid (salt) and the preparation thereof. It was the objective to provide a biologically degradable superabsorber with improved performance. This problem has been solved by a method for preparing a water-absorbing polyglutamic acid (salt), comprising the of:
i) providing an aqueous mixture of a non-crosslinked polyglutamic acid (salt) and at least one crosslinker,
ii) crosslinking the non-crosslinked polyglutamic acid (salt) to obtain a crosslinked polyglutamic acid (salt)-gel,
iii) drying the crosslinked polyglutamic acid (salt)-gel to obtain a dried crosslinked water-absorbing polyglutamic acid (salt),
iv) post-crosslinking the dried crosslinked water-absorbing polyglutamic acid (salt) to obtain the water-absorbing polyglutamic acid (salt).

EP 4 389 797 A1

**Description**

The present invention relates to a biologically degradable superabsorber based on polyglutamic acid (salt) and the preparation thereof.

"Superabsorbent" or "superabsorber" is a common commercial term referring to polymeric particles capable for absorbing huge amounts of water without releasing it under pressure. Other common terms are "super-absorbent material" (SAM), "super-absorbent polymers" (SAP) or "absorbent gelling material" (AGM).

When absorbing water, the superabsorbent swells impetuously; the water is caged within its polymeric network so that the water-laden superabsorbent forms a hydrogel. Likewise water, superabsorbents do absorb saline and different kinds of body fluids. Due to this capacity, superabsorbents serve as a key ingredient for personal hygiene products like baby diapers, feminine care products and incontinence articles.

Most of the super-absorbent materials used in hygiene articles today, which are capable of absorbing large quantities of liquids, among them, water and body fluids such as urine, in a short period of time are primarily based upon slightly crosslinked synthetic polymers. These include, for example, polymers and co-polymers based upon acrylic acid or acrylamide, which are traditionally not based upon renewable materials and are insufficiently or not at all biologically degradable resulting in the need for incineration or landfilling.

Attempts to replace the afore-mentioned polymers and co-polymers with biological degradable and hence sustainable polymers have been undertaken. One of the focuses lied upon the use of polysaccharides. A challenge entailed with the raw materials for the production of superabsorbers based upon polysaccharides is, however, that the raw material is frequently water-soluble and must be converted into the water-insoluble form, in order to be able to use them as superabsorbers in various applications.

For example, EP 0 538 904 A1 and U.S. Pat. No. 5,247,072 describe superabsorbers based upon carboxyalkylpolysaccharides. The process involves a thermal crosslinking step which is very sensitive to small changes of the pH value, the temperature of the reaction duration, absorbers with widely varying absorption properties are obtained with a tendency of shortened storage time duration.

In the processes known from the prior art for crosslinking of polysaccharides, however, besides the partially low aging stability, it is observed that the homogeneous crosslinking of the polysaccharides hinders the biodegradability of the absorber, since the accessibility for microorganisms is reduced by the restricted swelling. Furthermore, in the crosslinking reactions known from the prior art, the enzymatic breakdown is inhibited by the additionally introduced substituents [Mehltretter et al., Journal of the American Oil Chemists Society, 47 (1970), pages 522-524].

Due to its bio-based and biodegradable properties, polyglutamic acid (PGA) and its use as superabsorbent material was also investigated. For instance, WO 2021/242936 reports the manufacture of a superabsorbent based upon polyglutamic acid which may be additionally modified. The superabsorbent, however, shows mediocre performance with respect to common parameters used to assess a superabsorber, such centrifugal retention capacity (CRC), absorption against pressure (AAP) and saline flow conductivity (SFC) and absorption speed (Vortex and FSR), especially after the post-crosslinking step. As a proper performance regarding said parameters is crucial for commercial success of superabsorbents employed in personal hygiene articles, actual use of PGA-based superabsorbents in commercial hygiene articles is not known yet.

It is thus the objective of the present invention to provide a biologically degradable superabsorber with improved performance. In particular, performance regarding centrifugal retention capacity (CRC), absorption against pressure (AAP), saline flow conductivity (SFC) and absorption speed (Vortex and FSR) shall be improved and well-balanced.

This object is solved by a method for producing a water-absorbing polyglutamic acid (salt), comprising the method steps of:

i) providing an aqueous mixture of a non-crosslinked polyglutamic acid (salt) and at least one crosslinker,

ii) crosslinking the non-crosslinked polyglutamic acid (salt) to obtain a crosslinked polyglutamic acid (salt)-gel,

iii) drying the crosslinked polyglutamic acid (salt)-gel to obtain a dried crosslinked water-absorbing polyglutamic acid (salt),

iv) post-crosslinking the dried crosslinked water-absorbing polyglutamic acid (salt) to obtain the water-absorbing polyglutamic acid (salt).

It has been found that - if produced according to inventive method - superabsorbents based on polyglutamic acid (salt) achieve better performance in regard to demands of users and manufacturers of personal hygiene articles. Since superabsorbents obtained according to inventive process are originating from polyglutamic acid, they are biodegradable.

"At least one" as used herein relates to one or more, i.e., 1, 2, 3, 4, 5, 6, 7, 8, 9 or more. With respect to a crosslinker, for example, the value relates to the crosslinkers and not to the absolute number of the crosslinker's molecules employed. Unless stated otherwise, "%" refers to "wt.%".

A contribution to achieving the object is made by a method for producing a water-absorbing polyglutamic acid (salt), comprising the method steps of i) providing an aqueous mixture of a non-crosslinked polyglutamic acid (salt) and at least one crosslinker, ii) crosslinking the non-crosslinked polyglutamic acid (salt) to obtain a crosslinked polyglutamic acid (salt)-gel, iii) drying the crosslinked polyglutamic acid (salt)-gel to obtain a dried crosslinked water-absorbing polyglutamic acid (salt), and iv) post-crosslinking the dried crosslinked water-absorbing polyglutamic acid (salt) to obtain the water-absorbing polyglutamic acid (salt) provides for a sustainable and biologically degradable water-absorbing polyglutamic acid (salt).

The employed non-crosslinked polyglutamic acid (salt) is partially neutralized. Preferably, at least 50 %, more preferably at least 70 wt.% and even more preferably 90 wt.% of the non-crosslinked of the non-crosslinked polyglutamic acid is neutralized and present as salt. The neutralization can be accomplished by using standard techniques and methods known by the skilled person. For example, an aqueous solution of sodium hydroxide can be used. Suitable cations are lithium, sodium, and potassium and preferably sodium, potassium, and a mixture thereof are selected. The non-crosslinked polyglutamic acid is selected from $\alpha$-polyglutamic acid and $\gamma$-polyglutamic acid, preferably $\gamma$-polyglutamic acid is selected.

"Aqueous mixture", as used herein, refers to a mixture comprising at least 40 wt.%, preferably at least 50 wt.%, more preferably at least 60 wt.%, and even more preferably 70 wt.% water based on total weight of the aqueous mixture.

In a preferred embodiment of the present invention, the non-crosslinked polyglutamic acid (salt) has a molecular weight of 300,000 Dalton to 3,000,000 Dalton.

The at least one crosslinker is selected from the group consisting of the class of glycidylether such as diglycidylether, triglycidylether, polyglycidylether containing 3 or more epoxy groups, diglcerol tetraglycidyl ether, dipentaerythritol tetraglycidyl ether, pentaerythritol polyglycidyl ether, sorbitol polyglycidylether, isosorbide glycidyl ethers, polyglycerol-3-glydidyl ether, or other aliphatic polyfunctional epoxides, 1,4-butanediol diglycidyl ether, 1,6-hexanediol diglycidyl ether, propylene glycol diglycidyl ether, di propylene glycol diglycidyl ether, trimethylolpropane triglycidyl ether, poly propylene glycol diglycidyl ether, polyglycidyl ethers of alkanepolyols, polyglycidyl ethers of poly(alkylene glycols), biobased sorbitol glydidyl ether e.g. ERISYS® GE-61 from CVC Thermoset Specialties, any type of cyclic and aromatic poly glycidylethers, or a combination of more than one crosslinker of the class of glycidyl ether. The employed at least one crosslinker can be based on the carbodiimide chemistry and can be selected from 1-ethyl-3-(3-dimethylaminopropyl)carbodiimide hydrochloride, dicyclohexylcarbodiimide or other carbodiimides containing compounds or a combination of carbodiimide functionality containing crosslinker with other crosslinkers e.g. crosslinker of the class of glycidyl ethers saccharides such as glucose, maltotriose or cyclodextrin in the presence of water-soluble carbodiimide containing compound. Further crosslinkers can be used such as water-soluble chitosan, polyethylene glycol or other organic poly alcohols, aryl azide or diazirine or other photoreactive chemical hetero bi-functional crosslinking agents acting as receptor-ligand-interaction complexes by a 2-step activation, ethylene glycol diglycidyl ether, polyethylene glycol diglycidyl ether and 1 ,4-butanediol diglycidyl ether. Preferably, a crosslinker selected from the group consisting of ethylene glycol diglycidyl ether, polyethylene glycol diglycidyl ether and 1 ,4-butanediol diglycidyl ether is used.

In an embodiment of the invention, the aqueous mixture contains a water-soluble polymer which can be selected from the group consisting of a water-soluble polymer derived from the ethylenically unsaturated monomers containing acid groups defined in WO 2004/037903 A2, which is introduced herewith as reference and thus forms part of the disclosure. Among these polymers are based on the monomers acrylic acid and methacrylic acid, acrylamides, and methacrylamides. Preferred (meth)acrylamides are, in addition to acrylamide and methacrylamide, alkyl-substituted (meth)acrylamides or aminoalkyl-substituted derivatives of (meth)acrylamide, such as N-methylol(meth)acrylamide, N,N-dimethylamino(meth)acrylamide, dimethyl-(meth)acrylamide or diethyl(meth)acrylamide. Possible vinylamides are, for example, N-vinylamides, N-vinylformamides, N-vinylacetamides, N-vinyl-N-methylacetamides, N-vinyl-N-methylformamides and vinylpyrrolidone. Further, the water-soluble polymer can be based on alkoxy-polyalkylene oxide(meth)acrylates, such as methoxypolyethylene glycol(meth)acrylates, acrylic acid esters and methacrylic acid esters, such as methyl(meth)acrylate, ethyl(meth)acrylate, propyl(meth)acrylate or butyl(meth)acrylate. These monomers can also include methyl polyethylene glycol allyl ether, vinyl acetate, styrene and isobutylene.

In addition, further biologically degradable polymers can be employed such as polysaccharide.

Preferably, the aqueous mixture comprises:

a) 10.0 wt.% to 50.0 wt.%, preferably 20.0 wt.% to 40.0 wt.%, and more preferably 30.0 wt.% polyglutamic acid (salt) based on the total weight of the aqueous mixture,
b) 0.1 wt.% to 4.0 wt.%, preferably 0.3 wt.% to 3.0 wt.%, and more preferably 0.5 wt.% to 2.0 wt.% of the at least one crosslinker based on the total weight of the aqueous mixture,
c) 0.0 wt.% to 10.0 wt.%, preferably 0.0 wt.% to 5.0 wt.%, and more preferably 0.1 to 2.5 wt.% of a water-soluble polymer, and
d) 30.0 wt.% to 90.0 wt.%, preferably 50.0 wt.% to 80.0 wt.%, and more preferably 60.0 wt.% to 70.0 wt.% water.

Preferably, the aqueous mixture comprises 25.0 wt.% to 50.0 wt.%, preferably 30.0 wt.% to 40.0 wt.%, and more preferably 33.3 wt.% of a mixture comprising the polyglutamic acid and the at least one crosslinker based on the total weight of the aqueous mixture.

The crosslinking in step ii) of the method according to the present invention is preferably conducted within 10 min to 50 min, preferably within 20 min to 40 min, and more preferably within 30 min. In a preferred embodiment, step ii) is conducted under atmospheric pressure. In another preferred embodiment, step ii) is conducted under agitation. Step ii) is preferably conducted in a kneader reactor. The kneader reactor can be a single shaft kneader reactor. In another embodiment, the kneader reactor has at least two kneading shafts. Kneader type reactors are equipped with agitating means for disintegrating the polymerizing material right in the reaction vessel.

If a kneader reactor is used, the specific energy input exercised by the kneader during step ii) amounts from 10 J/g to 100 J/g, based on the mass of aqueous mixture. Compared to kneading of polymerization mixtures for conventional SAP based on acrylic acid, such specific energy input is low.

In a preferred embodiment, step ii) comprises a first stage and a second stage. The first stage is conducted under maximum agitation and the second stage at 20 % to 60 %, preferably 30 % to 50 % and more preferably 40 % of the maximum agitation. The time duration of the first stage is of between 50 % to 82 %, preferably 58 % to 76 %, and more preferably 66 % of the total time duration of step ii). The two stages ensure homogeneous reaction conditions of the processed material and has a beneficial effect on the obtained water-absorbing polyglutamic acid (salt). This method step is preferably conducted at a temperature of at least 80 °C, more preferably at least 90 °C, and even more preferably of at least 100 °C. The reaction vessel is ideally pre-heated to a specific temperature before the aqueous mixture is filled into the reaction vessel. A temperature of 110 °C is preferred.

Alternatively, to a kneader reactor, a reactor without agitating organs may be used, for example a belt reactor. Even in such case, crosslinked polyglutamic acid (salt)-gel shall be disintegrated before drying. This enhances the drying efficiency. However, gel obtained from a kneader reactor may be disintegrated before drying as well.

The disintegrating of the gel is performed by means of an extruder or a chopper or a mincer.

If such equipment is used for disintegrating the gel, the specific energy input exercised by the extruder or chopper or mincer during disintegration amounts from 10 J/g to 100 J/g, based on the mass of the crosslinked polyglutamic acid (salt)-gel.

The drying step iii) of the method is by means known in the art. Particularly, the gel can be dried in the form of a bed drier, or a fluidized bed drier or by using a microwave or convection drying. The drying in step iii) is preferably conducted for 5 min to 15 min, more preferably for 10 min at a temperature of between 100 °C to 160 °C, preferably between 115 °C to 145 °C, and more preferably at a temperature of 130 °C. Shorter drying time and/or lower drying temperature leads to wet material, whereas longer drying time and/or higher drying temperature results in undesired CRC loss and AAP loss of the water-absorbing polyglutamic acid (salt). Lower gel amounts allow a higher heat up rate and so on a better-balanced relation of AAP and CRC.

In case a bed drier is used, the crosslinked polyglutamic acid-gel is turned by 180 ° after 40 % to 60 %, preferably after 50 % of the drying step is conducted.

For drying the crosslinked polyglutamic acid-gel in step iii), the hot air stream used in the dryer shall have a velocity of 4 m/s to 7 m/s, preferably of 5.5 m/s.

According to a preferred embodiment the dried polyglutamic acid-gel is subject to a grinding step and a sieving step prior to step iv).

Preferably the sieving apparatus used in the sieving step shall have at least two sieves, the first sieve has a mesh size of 850 μm and the second mesh size of 150 μm.

The post-crosslinker used in step iv) is preferably selected from the group consisting of polyols, for example ethyleneglycol, polyethyleneglycols such as diethyleneglycol, triethyleneglycol and tetraethyleneglycol, propyleneglycol, polypropyleneglycols such as dipropyleneglycol, tripropyleneglycol or tetrapropyleneglycol, 1,3-butanediol, 1,4-butanediol, 1,5-pentanediol, 2,4-pentanediol, 1,6-hexanediol, 2,5-hexanediol, glycerine, polyglycerin, trimethylolpropane, polyoxypropylene, oxyethylene-oxypropylene-block copolymer, sorbitan-fatty acid esters, polyoxyethylenesorbitan-fatty acid esters, pentaerythritol, polyvinylalcohol and sorbitol, aminoalcohols, for example ethanolamine, diethanolamine, triethanolamine or propanolamine, polyamine compounds, for example ethylenediamine, diethylenetriamine, triethylenetetraamine, tetraethylenepentaamine or pentaethylenehexaamine, polyglycidyl ether compounds such as ethyleneglycoldiglycidyl ether, polyethyleneglycoldiglycidyl ether, glycerinediglycidyl ether, glycerinepolyglycidyl ether, pentaerithritolpolyglycidyl ether, propyleneglycoldiglycidyl ether, polypropyleneglycoldiglycidyl ether, neopentylglycoldiglycidyl ether, hexanediolglycidyl ether, trimethylolpropanepolyglycidyl ether, sorbitolpolyglycidyl ether, phthalic acid diglycidyl ester, adipinic acid diglycidyl ether, 1,4-phenylenebis(2-oxazoline), glycidol, polyisocyanates, preferably diisocyanates such as 2,4-toluenediioscyanate and hexamethylenediisocyanate, polyaziridine compounds such as 2,2-bishydroxymethylbutanol-tris[3-(1-aziridinyl)propionate], 1,6-hexa-methyl-enediethyleneurea and diphenylmethane-bis-4,4'-N,N'-diethyleneurea, halogen epoxides for example epichloro- and epibromohydrin and alpha-methylepichlorohydrin, alkylenecarbonates such as 1,3-dioxolane-2-one (ethylene carbonate), 4-methyl-1,3-dioxolane-2-one (propylene carbonate), 4,5-dimethyl-1,3-dioxolane-2-one, 4,4-dimethyl-1,3-dioxolane-2-one, 4-ethyl-1,3-dioxolane-2-one, 4-hydroxymethyl-1,3-dioxolane-2-one, 1,3-dioxane-2-one, 4-methyl-1,3-dioxane-2-one, 4,6-dimethyl-1,3-dioxane-2-one, 1,3-dioxolane-2-one, poly-1,3-dioxolane-2-on, polyquaternary amines such as condensation products from dimethylamines and epichlorohydrin. Further preferred surface crosslinkers are in addition polyoxazolines such as 1,2-ethylenebisoxazoline, crosslinkers with silane groups such as γ-glycidooxypropyltrimethoxysilane and γ-aminopropyltrimethoxysilane, oxazolidinones such as 2-oxazolidinone, bis- and poly-2-oxazolidinone and diglycolsilicates. According to yet another preferred embodiment, water is used as post-crosslinker in step iv).

0.10 wt.% to 2.00 wt.% of the post-crosslinker is regularly used, preferably 0.25 wt.% to 1.40 wt.%, and more preferably 0.50 wt.% to 0.80 wt.% based on the total amount of the dried water-absorbing polyglutamic acid (salt).

As mentioned above, the at least one post-crosslinker used in step iv) is preferably water. Even more preferably, step iv) is conducted in the absence of an organic compound in the form of organic solvents or crosslinkers or post-crosslinkers. In this context, "absence of an organic compound" refers to the intended addition of the organic compounds with the purpose of having an impact on the method step iv). In a preferred embodiment, 1.0 wt.% to 5.0 wt.%, more preferably 2.0 wt.% to 4.0 wt.%, and even more preferably 3.0 wt.% water is used based on the total amount of the dried crosslinked water-absorbing polyglutamic acid (salt). Without wishing to be bond to a specific theory, the water assists the post-crosslinking, although water cannot be considered to be as a chemical post-crosslinker.

The present invention also relates to a water-absorbing polyglutamic acid (salt) obtainable by a method described herein. Preferably, the water-absorbing polyglutamic acid (salt) possess a particulate form and/or the water-absorbing polyglutamic acid (salt) possess a particle size distribution of between 150 μm to 850 μm.

Preferably, the water-absorbing polyglutamic acid (salt) obtainable according to present process achieves at least one of the following performance parameters:

- an absorbency against pressure of 4.83 kPa (0.7psi) of more than 9 and less than 24 g/g, preferably less than 26 g/g, as measured as defined in the description;
- a saline flow conductivity of more than 0 and less than $150 \times 10^{-7} \cdot cm^3 \cdot s \cdot g^{-1}$, preferably more than 10 and less than $100 \times 10^{-7} \cdot cm^3 \cdot s \cdot g^{-1}$ as measured as defined in the description;
- a centrifugal retention capacity of more than 20 and less than 46 g/g, preferably more than 25 and less than 42 g/g as measured as defined in the description;
- a free swell rate of more than 0.5 and less than 1.5 g/g*s, preferably 0.7 and less than 1.2 g/g*s as measured as defined in the description;
- a Vortex of less than 50 and more than 10 seconds, preferably 30 and more than 15 seconds as measured as defined in the description.

Said performance parameters are frequently used for qualifying superabsorbents for use in personal hygiene articles. Cited values are resulting from inventive preparation method.

Yet another object of the present invention is an article comprising the water-absorbing polyglutamic acid with at least one of the performance parameters or which is obtainable by the inventive method.

Said article is preferably a personal hygiene article such as a diaper, a sanitary towel, or a napkin. Besides that, the article may be a wound covering. Further applications of the inventive water-absorbing polyglutamic acid (salt) include medicine/pharmaceutical applications where it can be used as or in biological glue, dental carriers, bone regeneration, cartilage regeneration, vaccine development scaffolds in tissue engineering, drug delivery system, and biological control agent. Further applications are agricultural, e.g. for improving the soil water availability, the use as biofertilizer, in wastewater treatment, as bio flocculant, in the food industry as thickener, oil reducing agent, flavoring agent, cryoprotectant, and in cosmetics, such as sunscreens, hair growth serums, anti-aging serums, mouth wash, and contact lens care solutions.

Test methods

Superabsorbents are usually purchased by manufacturers of personal hygiene products. For purchase decision, the overall performance profile of a superabsorbent is crucial. The required performance profile strongly depends on the type and purpose of the hygiene article. During the last decades, a bunch of performance parameters has been established by the market participants for comparing the suitability of different SAP qualities for an intended purpose. Some of those performance parameters are standardized by independent authorities, others are defined by certain manufacturers of hygiene products for their individual needs only. Actually, both types of performance parameters are of importance within the real-life SAP market. Besides that, the patent literature is jam-packed with parameters which do not have any impact outside a certain patent right at all.

For the present invention, the following performance parameters are relevant:

Centrifugal retention capacity (CRC). One of the oldest performance parameters for SAP. Refers to the capacity for absorbing urine. Unit is g/g or [-]. The higher the value, the higher the capacity. As the test method formally uses a teabag to be filled with SAP, this value has been often referred as "teabag-capacity". The test method includes centrifugation the loaded probe. However, chosen rotation speed and therefore the centrifugal force G differs from time to time. Hence the value is only reliable with given speed of rotation or G force. The method has been standardized by business organization EDANA as Standard Test WSP 241.2 (05).

Absorbency under a pressure (AUP). Refers to the capacity of absorbing urine when subjected to an external pressure. Unit is g/g or [-]. The higher the value, the higher the capacity. The pressure applied on the probe differs. Some ones use a pressure of 4.83 kPa (=0.7 psi, thus named AUP 0.7), others prefer 2.07 kPa (=0.3 psi, named AUP 0.3 accordingly). Hence, the value is informative only if applied pressure is given. Herein, AUP has been measured at 4.83 kPa. Sometimes, the AUP value is called "Absorbency under Load (AUL)" or "Absorption against pressure (AAP)" as well. The method has been standardized by business organization EDANA as Standard Test WSP 242.2 (05).

Saline flow conductivity (SFC). To maintain transportation of body fluids within the hygiene article once a first contact with body fluid has occurred, gel still needs to have ability to conduct fluids. This property is named permeability. A frequently used performance parameter for measuring permeability of a superabsorbent is saline flow conductivity (SFC). This parameter and related test method has been developed by a major manufacturer of hygiene articles. A definition of SFC test method is disclosed in WO-A-95/26209, pages 69 to 75, although 1.5 g superabsorbent was used instead of 0.9 g.

Free swell rate (FSR). This parameter relates to the speed of swelling. 1.00 g (= W1 ) of the dry water-absorbing material is weighed into a 25 ml glass beaker and is uniformly distributed on the base of the glass beaker. 20 ml of a 0.9% by weight sodium chloride solution are then dispensed into a second glass beaker, the contents of this beaker are rapidly added to the first beaker and a stopwatch is started. As soon as the last drop of salt solution is absorbed, confirmed by the disappearance of the reflection on the liquid surface, the stopwatch is stopped. The exact amount of liquid poured from the second beaker and absorbed by the polymer in the first beaker is accurately determined by weighing back the second beaker (=W2). The time needed for the absorption, which was measured with the stopwatch, is denoted t. The disappearance of the last drop of liquid on the surface is defined as time t. The free swell rate (FSR) is calculated as follows:

$$FSR \ [g/gs] = W2/(W1 {*} t)$$

When the moisture content of the hydrogel-forming polymer is more than 3% by weight, however, the weight W1 must be corrected for this moisture content. The units on FSF are g/gsec. The higher the value the faster the absorption.

Yet another parameter for measuring the speed of a superabsorbent is vortex time. The vortex time of the super absorbent polymer is measured in the amount of time in seconds until the vortex disappeared after adding 2 g of a super absorbent polymer to 50 mL of physiological saline solution and then stirring the mixture at 600 rpm. A 100 mL glass beaker (Pyrex #1060-100 or Fisher Brand #FB-102-100) was used together with a magnetic stirring rod (size 38.4 mm $\times$ 8 mm). The saline temperature was set to be between 24 °C and 25 °C and the temperature of the room was above 21 °C. The humidity was set to be within the limitations of EDANA Standard Test WSP 241.2 (05).

As the definition of performance parameters changes overtime, comparing certain values is not possible without considering the precise measurement method before its time horizon.

Invention shall now more elucidated by means of following examples.

**Example 1**

Preparation of the monomer solution for experiments on lab scale

For the preparation of the monomer solution 66,6 parts of DI-water and from 0,5 to 2% based on PGA of a diglycidyl ether type crosslinker were carefully mixed at room temperature. The active substance or total solid of the gel is so of 33,3% of the total mixture.

Used crosslinkers (in the respective trials):

- Ethylene glycol diglycidyl ether (EDGDE)
- Polyethylene glycol diglycidyl ether (poly-EGDGE)
- 1,4-butandiol diglycidyl ether (BDDGE) to optimize the quality

Batch-Polymerization in the kneader reactor

The "polymerization" reactions were conducted in a single shaft kneader reactor of the type Discotherm Batch, DTB1.5, obtained by List AG Switzerland, with a total volume of 3,1 liters. The reactor is equipped with a jacket for heating and cooling, a vacuum system, a nitrogen supply system, and a reactor control unit.

A portion of 1000 g water/crosslinker solution prepared as described above, were filled into the reactor through the dome and thereafter 500 g of a PGA in solid form is added under full speed agitation. The reactor was previously heated up to more than 110°C. The pressure of the vessel is the atmospheric pressure. The reactor is kept open and at the previous mentioned temperature over the entire reaction.

The agitation is set to the maximum (100 % of the potty, 65 rpm) for 20 minutes to allow the PGA to dissolve completely and the mixture to reach the wished temperature. After this, the agitator is set to 40 %, e.g., 26 rpm, for additional 10 minutes. Thereafter, the endplate of the reactor is removed to discharge the free flowing and granulated gel from the reactor. The obtained polymer is further processed as described above.

Important information to achieve the best product quality:

- Reduced toxicity by using poly-EGDGE or equivalent
- Active substance 33,3 %
- Temperature of minimum 110 °C
- Fast agitation for the two third of the reaction and then reduction to not over knead the gel
- Fill grade of the reactor of minimum 50 %

Gel dryer

On lab scale, gel drying experiments are performed in a batch-operated Fluidized Bed Drier, type CTL (delivered be Allgaier-Werke KG, Ulmerstr. 75, D-7336 Uhingen). This drier is equipped with a conically shaped fluidization chamber having on the bottom side the same plate than the plate belt dryers of the plant, diameter of 20 cm, a ventilator, an air heater, a fresh air and an exhaust air filter with automatic de-dusting and a control box. The air stream is flowing bottom-up and is not circulated.

The gel to be dried is placed on the plate, the gel bed having a thickness of about 5 cm and is dried at 130°C by the hot-air stream which is forced through the gel bed for 10 minutes. After this the material "pizza" is turned by 180°, to allow a homogeneous drying on both side for further 5 minutes. This drier can simulate the plant-scale belt drier.

Important information to reach the best product quality:

- Shorter residence time leads to wet material
- Longer residence time leads to an overdrying with CRC and AAP loss
- Lower drying temperature leads to wet material
- Higher drying temperature leads to an overdrying with CRC and AAP loss
- Lower gel amounts allow a higher heat up rate and so on a better CRC-AAP ration development

Gel Sizing and drying, grinding and sieving in the lab

The granulated gel obtained from the "polymerization" in the DTB 1,5l reactor is extruded in a kitchen type meat mincer equipped with a die plate having holes diameter of 6 mm. A portion of 600 grams of the extruded gel is then placed in the fluidization chamber of the dryer and is dried in the hot air stream at 5,5 m/s air inlet velocity at 130°C for 15 minutes (10+5). The dry polymer obtained is ground in a Bauermeister lab roll mill and sieved in a Retsch sieve tower equipped with the sieves having mesh sizes of 850 and 150 microns, obtaining the particle size fraction for analysis.

Preparation of Surface Crosslinked / Posttreated Superabsorber

The dry polymers (precursors) obtained after drying grinding and sieving were surface treated (surface crosslinked) to improve their properties.

To prepare the surface-crosslinking solution 0.5-0.8% crosslinker depending on the molecular weight and the reactivity, was dissolved in 3% demineralized water, each based on 100g of solid precursor material.

As crosslinker Ethylene glycol diglycidyl ether (EGDGE) and 1,4-butanediol diglycidyl ether (BDDGE) were used.

60g of the precursor material was coated in a household Krups blender cup with the aforementioned amount of surface-crosslinking solution while strong stirring with a Krups 3 Mix 7000 mixer by means of a syringe with 0.4mm cannula and stirred for another minute.

Some precursor samples were only coated with 3% water in the same way.

The coated precursor was divided into 3 portions and heated in a circulating air-drying oven Heraeus UT6120 for 20-60 minutes at 150 ° C.

The surface crosslinked polymers thus obtained were sieved with a Retsch sieve having a mesh size of 850 microns to remove agglomerates formed by the coating.

**Example 2**

Preparation of the water/crosslinker solution

For the preparation of the monomer solution 1000 g of DI-water and from 0,25 g to 15 g of a diglycidyl ether type crosslinker were mixed at room temperature. Most preferably, 10g of a crosslinker solution (not active substance) were mixed in 1000 g of DI-water, in a beaker with the help of a magnetic stirrer.

Used crosslinkers:

- Ethylene glycol diglycidyl ether (EDGDE)
- Polyethylene glycol diglycidyl ether (p-EGDGE)
- 1,4-butandiol diglycidyl ether (1,4-BDDGE)

Co-polymerization in the kneader reactor

The crosslinking/copolymerization reactions were conducted in a single shaft kneader reactor of the type Discotherm Batch, DTB1.5, obtained by List AG Switzerland, with a total volume of 3,1 liters. The reactor is equipped with a jacket for heating and cooling, a vacuum system, a nitrogen supply system, and a reactor control unit.

The water/crosslinker solution prepared as described above, was filled into the kneader reactor through the dome and thereafter 500 g of a PGA (ex Lubon Industry Co. Ltd., China, MW = 700,000 Da; or ex Bonding Chemical, USA, MW = 1,100,000 Da) in solid form is added under full speed agitation (65 rpm). The reactor was previously heated up to more than 110 °C (set temperature of the heating device: 116 °C). The temperature was measured was situated in the jacket of the kneader reactor.

The pressure of the vessel is the atmospheric pressure. The reactor is kept open and at the previous mentioned temperature over the entire reaction.

The agitation is set to the maximum (100 % of the potty, 65 rpm) for 20 minutes to allow the PGA to dissolve completely and the mixture to reach the desired temperature. After this, the agitator is set to 40 %, 26 rpm, for additional 10 minutes. Thereafter, the endplate of the reactor is removed to discharge the free flowing and granulated gel from the reactor. The obtained polymer is further processed as described above.

The gel was chopped in a Mado meat mincer MEW 710-R70 with a hole plate having a 6 mm diameter.

Important information to achieve the best product quality:

- Reduced toxicity by using poly-EGDGE or equivalent
- Active substance 33.3 wt.%
- Kneader jacket: Temperature of minimum 110 °C
- Fast agitation for the two third of the reaction and then reduction to not over knead the gel
- Filling degree of the reactor of minimum 50 %

Gel drier

On lab scale, gel drying experiments are performed in a batch-operated Fluidized Bed Drier, type CTL (delivered be Allgaier-Werke KG, Ulmerstr. 75, D-7336 Uhingen). This drier is equipped with a conically shaped fluidization chamber having on the bottom side the same plate than the plate belt dryers of the plant, diameter of 20 cm, a ventilator, an air heater, a fresh air and an exhaust air filter with automatic de-dusting and a control box. The air stream is flowing bottom-up and is not circulated.

The gel to be dried is placed on the plate, the gel bed having a thickness of about 5 cm and is dried at 130 °C by the hot-air stream which is forced through the gel bed for 10 minutes. After this the material touch dry material is turned upside down, to allow a homogeneous drying on both side for further 5 minutes. This drier is capable of simulating the plant-scale belt drier. Moisture content at the end of drying step was 5 to 8 %; measured by means of a halogen moisture analyser.

Important information to achieve the best product quality:

- Shorter residence time leads to wet material

- Longer residence time leads to an overdrying with CRC and AAP loss
- Lower drying temperature leads to wet material
- Higher drying temperature leads to an overdrying with CRC and AAP loss
- Lower gel amounts allow a higher heat up rate and so on a better CRC-AAP ration development

Gel Sizing and drying, grinding and sieving in the lab

The granulated gel obtained from the polymerization in the DTB 1,5l kneader reactor is extruded in a kitchen type meat mincer equipped with a die plate having holes diameter of 6 mm. A portion of 600 grams of the extruded gel is then placed in the fluidization chamber of the dryer and is dried in the hot air stream at 5,5 m/s air inlet velocity at 130°C for 15 minutes (10+5). The dry polymer obtained is ground in a Bauermeister lab roll mill and sieved in a Retsch sieve tower equipped with the sieves having mesh sizes of 850 and 150 microns, obtaining the particle size fraction for analysis.

**Results**

The performance data of water-absorbing polyglutamic acid (salt) prepared according to examples 1 and 2 are shown in the tables. Table 1 displays the data of precursor product obtained after gel drying while table 2 shows performance data of the final product after post-crosslinking.

Table 1:    Data of precursor product after gel drying
Table 2:    Data of final product after post-crosslinking

**Table 1.** Data of precursor product after gel drying

| Lab ID | crosslinker | Amount crosslinker (relation to PGA) | Temperature Heat jacket | Drying temp | Gel amount | CRC | AAP 0.7 psi | SFC | Vortex | FSR |
|---|---|---|---|---|---|---|---|---|---|---|
| [#] | [-] | [wt.%] | [°C] | [°C] | [g] | [g/g] | [g/g] | [$10^{-7}$ cm$^3$s/g] | [s] | [g/g/s] |
| 9.1 | p-EGDGE | 2 | 105 | 140 | 600 | 25.8 | 16.1 | 8 | 19 | 0.82 |
| 9.2 | p-EGDGE | 2 | 105 | 130 | 600 | 26.3 | 14.2 | 11 | 29 | 0.69 |
| 22.1 | p-EGDGE | 2 | 115 | 130 | 600 | 32.9 | 9.1 | | | |
| 23.1 | p-EGDGE | 2 | 115 | 130 | 600 | 25.4 | 21.1 | | | |
| 23.2 | 1,4-BDDGE | 2 | 115 | 130 | 600 | 25.6 | 21.5 | | | |
| 24 | 1,4-BDDGE | 2 | 115 | 130 | 700 | 27.4 | 21.1 | | | |
| 27.2 | 1,4-BDDGE | 2 | 115 | 130 | 600 | 24.2 | 21.4 | | | |

Table 2. Data of final product after post-crosslinking

| PX-ID | Amount EGDGE | Amount 1,4-BDDGE | Amount water | Amount Isopropanol | PX condition | CRC | AAP 0.7 psi | SFC | Vortex | FSR |
|---|---|---|---|---|---|---|---|---|---|---|
| [#] | [g] | [g] | [g] | [g] | [min / °C] | [g/g] | [g/g] | [$10^{-7}$ cm$^3$s/g] | [s] | [g/g/s] |
| 9.1-A | 0.5 | - | 3 | 1 | 20 / 150 | 25.8 | 21.1 | 26 | 28 | 0.78 |
| 9.1-B | 0.5 | - | 3 | 1 | 40 / 150 | 27.3 | 21.2 | | | |
| 9.1-C | 0.5 | - | 3 | 1 | 60 / 150 | 28.0 | 20.9 | 19 | 25 | 0.79 |
| 9.2-A | - | 0.8 | 3 | - | 20 / 150 | 26.2 | 21.5 | 27 | 30 | 0.70 |
| 9.2-B | - | 0.8 | 3 | - | 40 / 150 | 27.2 | 21.5 | | | |
| 9.2-C | - | 0.8 | 3 | - | 60 / 150 | 28.8 | 21.2 | 20 | 29 | 0.73 |
| 22.1-A | - | 0.8 | 3 | - | 20 / 150 | 38.1 | 11.3 | 0 | 24 | 0.80 |
| 22.1-B | - | 0.8 | 3 | - | 40 / 150 | 43.7 | 10.2 | | | |
| 22.1-C | - | 0.8 | 3 | - | 60 / 150 | 45.8 | 9.6 | 0 | 22 | 0.88 |
| 23.1-A | - | - | 3 | - | 20 / 150 | 29.4 | 23.2 | 38 | 19 | 1.02 |
| 23.1-B | - | - | 3 | - | 40 / 150 | 32.2 | 22.8 | | | |
| 23.1-C | - | - | 3 | - | 60 / 150 | 33.6 | 23.3 | 17 | 19 | 1.11 |
| 23.2-C1 | - | - | 3 | - | 60 / 150 | 31.2 | 22.4 | | | |
| 23.2-C2 | - | - | 3 | - | 60 / 150 | 32.2 | 23.2 | | | |
| 23.2-C1+2 | - | - | 3 | - | 60 / 150 | 31.7 | 22.8 | 26 | 15 | 1.00 |
| 24-A | - | - | 3 | - | 20 / 150 | 32.7 | 20.9 | 14 | 24 | 0.86 |
| 24-B | - | - | 3 | - | 40 / 150 | 37.3 | 19.4 | | | |
| 24-C | - | - | 3 | - | 60 / 150 | 38.6 | 16.6 | | | |
| 24-D | - | 0.8 | 3 | - | 20 / 150 | 32.5 | 21.7 | 14 | 21 | 0.90 |
| 24-E | - | 0.8 | 3 | - | 40 / 150 | 36.6 | 20.2 | | | |
| 24-F | - | 0.8 | 3 | - | 60 / 150 | 38.0 | 17.8 | | | |
| 27.2-A | - | - | 3 | - | 20 / 150 | 27.0 | 23.2 | 65 | 23 | 0.77 |
| 27.2-B | - | - | 3 | - | 40 / 150 | 28.3 | 22.8 | 54 | 21 | 0.80 |

(continued)

| PX-ID | Amount EGDGE | Amount 1,4-BDDGE | Amount water | Amount Isopropanol | PX condition | CRC | AAP 0.7 psi | SFC | Vortex | FSR |
|---|---|---|---|---|---|---|---|---|---|---|
| [#] | [g] | [g] | [g] | [g] | [min / °C] | [g/g] | [g/g] | [$10^{-7}$ cm$^3$s/g] | [s] | [g/g/s] |
| 27.2-C | - | - | 3 | - | 60 / 150 | 28.9 | 23.0 | 48 | 24 | 0.81 |
| PX = post-crosslinking | | | | | | | | | | |

Claims

1. A method for producing a water-absorbing polyglutamic acid (salt), comprising the method steps of:

    i) providing an aqueous mixture of a non-crosslinked polyglutamic acid (salt) and at least one crosslinker,
    ii) crosslinking the non-crosslinked polyglutamic acid (salt) to obtain a crosslinked polyglutamic acid (salt)-gel,
    iii) drying the crosslinked polyglutamic acid (salt)-gel to obtain a dried crosslinked water-absorbing polyglutamic acid (salt),
    iv) post-crosslinking the dried crosslinked water-absorbing polyglutamic acid (salt) to obtain the water-absorbing polyglutamic acid (salt).

2. The method according to claim 1, **characterized in, that**
the non-crosslinked polyglutamic acid (salt) has a molecular weight of 300,000 Dalton to 3,000,000 Dalton.

3. The method according to claim 1 or 2, **characterized in that**
the crosslinker is selected from the group consisting of the class of glycidylether such as diglycidylether, triglycidylether, polyglycidylether containing 3 or more epoxy groups, diglcerol tetraglycidyl ether, dipentaerythritol tetraglycidyl ether, pentaerythritol polyglycidyl ether, sorbitol polyglycidylether, isosorbide glycidyl ethers, polyglycerol-3-glydidyl ether, or other aliphatic polyfunctional epoxides, 1,4-butanediol diglycidyl ether, 1,6-hexanediol diglycidyl ether, propylene glycol diglycidyl ether, di propylene glycol diglycidyl ether, trimethylolpropane triglycidyl ether, poly propylene glycol diglycidyl ether, polyglycidyl ethers of alkanepolyols, polyglycidyl ethers of poly(alkylene glycols), biobased sorbitol glydidyl ether, cyclic and aromatic poly glycidylethers, or a combination of more than one crosslinker of the class of glycidyl ether, the class of carbodiimide such as 1-ethyl-3-(3-dimethylaminopropyl)carbodiimide hydrochloride, dicyclohexylcarbodiimide or other carbodiimides containing compounds or a combination of carbodiimide functionality containing crosslinker with other crosslinkers e.g. crosslinker of the class of glycidyl ethers saccharides such as glucose, maltotriose or cyclodextrin in the presence of water-soluble carbodiimide containing compound, other crosslinkers such as water-soluble chitosan, polyethylene glycol or other organic poly alcohols, aryl azide or diazirine or other photoreactive chemical hetero bi-functional crosslinking agents acting as receptor-ligand-interaction complexes by a 2-step activation, ethylene glycol diglycidyl ether, polyethylene glycol diglycidyl ether and 1,4-butanediol diglycidyl ether, and preferably from the group consisting of ethylene glycol diglycidyl ether, polyethylene glycol diglycidyl ether and 1,4-butanediol diglycidyl ether.

4. The method according to claim 3, **characterized in that**
the at least one crosslinker is selected from the group consisting of ethylene glycol diglycidyl ether, polyethylene glycol diglycidyl ether and 1,4-butanediol diglycidyl ether.

5. The method according to any one of the claims 1 to 4, **characterized in that** the aqueous mixture comprises:

    a) 10.0 wt.% to 50.0 wt.%, preferably 20.0 wt.% to 40.0 wt.%, and more preferably 30.0 wt.% polyglutamic acid (salt) based on the total weight of the aqueous mixture,
    b) 0.1 wt.% to 4.0 wt.%, preferably 0.3 wt.% to 3.0 wt.%, and more preferably 0.5 wt.% to 2.0 wt.% of the at least one crosslinker based on the total weight of the aqueous mixture,
    c) 0.0 wt.% to 10.0 wt.%, preferably 0.0 wt.% to 5.0 wt.%, and more preferably 0.1 to 2.5 wt.% of a water-soluble polymer, and
    d) 30.0 wt.% to 90.0 wt.%, preferably 50.0 wt.% to 80.0 wt.%, and more preferably 60.0 wt.% to 70.0 wt.% water.

6. The method according to any one of the claims 1 to 5, **characterized in that** the aqueous mixture comprises 25.0 wt.% to 50.0 wt.%, preferably 30.0 wt.% to 40.0 wt.%, and more preferably 33.3 wt.% of a mixture comprising the polyglutamic acid (salt) and the at least one crosslinker based on the total weight of the aqueous mixture.

7. The method according to any one of the claims 1 to 6, **characterized in that**

    step ii) is conducted within 10 min to 50 min, preferably within 20 min to 40 min, and more preferably within 30 min; and/or
    step ii) is conducted under atmospheric pressure; and/or
    step ii) is conducted under agitation; and/or
    step ii) is conducted in a kneader reactor.

8. The method according to claim 7, whereby step ii) is conducted in a kneader reactor **characterized in that** the specific energy input exercised by the kneader during step ii) amounts from 10 J/g to 100 J/g, based on the mass of aqueous mixture.

9. The method according to claim 7 or 8, **characterized in that** the time duration of the first stage is of between 50 % to 82 %, preferably 58 % to 76 %, and more preferably 66 % of the total time duration of step ii).

10. The method according to any one of the claims 1 to 9, **characterized in that** the crosslinked polyglutamic acid (salt)-gel is disintegrated before drying.

11. The method according to claim 10, **characterized in that** disintegrating is performed by an extruder or chopper or mincer.

12. The method according to claim 11, **characterized in that** the specific energy input exercised by the extruder or chopper or mincer during disintegration amounts from 10 J/g to 100 J/g, based on the mass of the crosslinked polyglutamic acid (salt)-gel.

13. The method according to any one of the claims 1 to 12, **characterized in that** step iii) is conducted in a fluidized bed drier or on a belt drier.

14. The method according to any one of the claims 1 to 13, **characterized in that** step iii) is conducted for 5 min to 15 min, preferably for 10 min at a temperature of between 100 °C to 160 °C, preferably between 115 °C to 145 °C, and more preferably at a temperature of 130 °C.

15. The method according to any one of claims 1 to 14, **characterized in that** the dried polyglutamic acid (salt)-gel is subject to a grinding step and a sieving step prior to step iv).

16. The method according to claim 15, **characterized in that**
a sieving apparatus is used in the sieving step, wherein the sieving apparatus has at least two sieves, the first sieve has a mesh size of 850 $\mu$m and the second mesh size of 150 $\mu$m.

17. The method according to any one of the claims 1 to 16, **characterized in that** in step iv) at least one post-crosslinker is used selected from the group consisting of polyols, for example ethyleneglycol, polyethyleneglycols such as diethyleneglycol, triethyleneglycol and tetraethyleneglycol, propyleneglycol, polypropyleneglycols such as dipropyleneglycol, tripropyleneglycol or tetrapropyleneglycol, 1,3-butanediol, 1,4-butanediol, 1,5-pentanediol, 2,4-pentanediol, 1,6-hexanediol, 2,5-hexanediol, glycerine, polyglycerin, trimethylolpropane, polyoxypropylene, oxyethyleneoxypropylene-block copolymer, sorbitan-fatty acid esters, polyoxyethylenesorbitan-fatty acid esters, pentaerythritol, polyvinylalcohol and sorbitol, aminoalcohols, for example ethanolamine, diethanolamine, triethanolamine or propanolamine, polyamine compounds, for example ethylenediamine, diethylenetriamine, triethylenetetraamine, tetraethylenepentaamine or pentaethylenehexaamine, polyglycidyl ether compounds such as ethyleneglycoldiglycidyl ether, polyethyleneglycoldiglycidyl ether, glycerinediglycidyl ether, glycerinepolyglycidyl ether, pentaerithritolpolyglycidyl ether, propyleneglycoldiglycidyl ether, polypropyleneglycoldiglycidyl ether, neopentylglycoldiglycidyl ether, hexanediolglycidyl ether, trimethylolpropanepolyglycidyl ether, sorbitolpolyglycidyl ether, phthalic acid diglycidyl ester, adipinic acid diglycidyl ether, 1,4-phenylenebis(2-oxazoline), glycidol, polyisocyanates, preferably diisocyanates such as 2,4-toluenediioscyanate and hexamethylenediisocyanate, polyaziridine compounds such as 2,2-bishydroxymethylbutanol-tris[3-(1-aziridinyl)propionate], 1,6-hexa¬methyl¬enediethyleneurea and diphenylmethane-bis-4,4'-N,N'-diethyleneurea, halogen epoxides for example epichloro- and epibromohydrin and alpha-methylepichlorohydrin, alkylenecarbonates such as 1,3-dioxolane-2-one (ethylene carbonate), 4-methyl-1,3-dioxolane-2-one (propylene carbonate), 4,5-dimethyl-1,3-dioxolane-2-one, 4,4-dimethyl-1,3-dioxolane-2-one, 4-ethyl-1,3-dioxolane-2-one, 4-hydroxymethyl-1,3-dioxolane-2-one, 1,3-dioxane-2-one, 4-methyl-1,3-dioxane-2-one, 4,6-dimethyl-1,3-dioxane-2-one, 1,3-dioxolane-2-one, poly-1,3-dioxolane-2-on, polyquaternary amines such as condensation products from dimethylamines and epichlorohydrin. Further preferred surface crosslinkers are in addition polyoxazolines such as 1,2-ethylenebisoxazoline, crosslinkers with silane groups such as γ-glycidooxypropyltrimethoxysilane and γ-aminopropyltrimethoxysilane, oxazolidinones such as 2-oxazolidinone, bis- and poly-2-oxazolidinone and diglycolsilicates, and water.

18. The method according to claim 17, **characterized in that**
the at least one post-crosslinker used in step iv) is selected from the group consisting of ethylene glycol diglycidyl ether, polyethylene glycol diglycidyl ether and 1,4-butanediol diglycidyl ether.

19. The method according to claim 17 or 18, **characterized in that**
0.10 wt.% to 2.00 wt.%, preferably 0.25 wt.% to 1.40 wt.%, and more preferably 0.50 wt.% to 0.80 wt.% of the post-crosslinker is used based on the total amount of the dried water-absorbing polyglutamic acid (salt).

20. The method according to any one of claims 1 to 19, **characterized in that** the at least one post-crosslinker used in step iv) is water.

21. The method according to claim 20, **characterized in that**
1.0 wt.% to 5.0 wt.%, preferably 2.0 wt.% to 4.0 wt.%, and more preferably 3.0 wt.% water is used as post-crosslinker, based on the total amount of the dried water-absorbing polyglutamic acid (salt).

22. A water-absorbing polyglutamic acid (salt) obtainable by a method according to any of the claims 1 to 21.

23. A water-absorbing polyglutamic acid (salt), **characterized in that** said water-absorbing polyglutamic acid (salt) possesses at least one of the following features:

• an absorbency against pressure of 4.83 kPa (0.7psi) of more than 9 and less than 24 g/g, preferably less than 26 g/g, as measured as defined in the description;
• a saline flow conductivity of more than 0 and less than $150 \times 10^{-7} \cdot cm^3 \cdot s \cdot g^{-1}$, preferably more than 10 and less than $100 \times 10^{-7} \cdot cm^3 \cdot s \cdot g^{-1}$ as measured as defined in the description;
• a centrifugal retention capacity of more than 20 and less than 46 g/g, preferably more than 25 and less than 42 g/g as measured as defined in the description;
• a free swell rate of more than 0.5 and less than 1.5 g/g*s, preferably 0.7 and less than 1.2 g/g*s as measured as defined in the description;
• a Vortex of less than 50 and more than 10 seconds, preferably 30 and more than 15 seconds as measured as defined in the description.

24. An article comprising the water-absorbing polyglutamic acid (salt) according to claim 22 or to claim 23.

25. The article according to claim 24, **characterized in that**
the article is or comprises a diaper, hygiene article, a sanitary towel, a napkin, a wound covering.

Europäisches
Patentamt

European
Patent Office

Office européen
des brevets

# EUROPEAN SEARCH REPORT

Application Number

EP 22 21 4780

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/242936 A1 (ZYMOCHEM INC [US]) 2 December 2021 (2021-12-02) * claims * * paragraph [0125] - paragraph [0143]; examples * * tables 3, 4, 9 * * paragraph [0066] * * paragraph [0108] * * paragraph [0082] * * paragraph [0091] * * paragraph [0094] * ----- | 1-25 | INV. C08G69/10 A61F13/49 C08G69/48 |
| X | JP 6 474251 B2 (LIVEDO CORP; NAGASE & CO LTD ET AL.) 27 February 2019 (2019-02-27) * claims * * examples * * tables 1, 2 * * paragraph [0053] * * paragraph [0041] * * paragraph [0043] * * paragraph [0083] * ----- | 1-25 | |

TECHNICAL FIELDS
SEARCHED      (IPC)

C08G
A61F
C08L

The present search report has been drawn up for all claims

| Place of search | Date of completion of the search | Examiner |
|---|---|---|
| Munich | 31 May 2023 | Mader, Margarita |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 21 4780

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2023

| Patent document cited in search report | | Publication date | Patent family member(s) | | Publication date |
|---|---|---|---|---|---|
| WO 2021242936 | A1 | 02-12-2021 | AU 2021279010 | A1 | 08-12-2022 |
| | | | BR 112022023972 | A2 | 07-02-2023 |
| | | | CA 3178411 | A1 | 02-12-2021 |
| | | | CN 115916901 | A | 04-04-2023 |
| | | | EP 4158049 | A1 | 05-04-2023 |
| | | | KR 20230035254 | A | 13-03-2023 |
| | | | US 2023058841 | A1 | 23-02-2023 |
| | | | WO 2021242936 | A1 | 02-12-2021 |
| JP 6474251 | B2 | 27-02-2019 | JP 6474251 | B2 | 27-02-2019 |
| | | | JP 2016121287 | A | 07-07-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0538904 A1
- US 5247072 A
- WO 2021242936 A
- WO 2004037903 A2
- WO 9526209 A

**Non-patent literature cited in the description**

- **MEHLTRETTER et al.** *Journal of the American Oil Chemists Society,* 1970, vol. 47, 522-524